Europäisches Patentamt

European Patent Office

Office européen des brevets

(11) Publication number: **0 442 663 A1**

## EUROPEAN PATENT APPLICATION

(21) Application number: 91300997.3

(22) Date of filing: 06.02.91

(51) Int. Cl.5: **A61K 33/24**, A61K 33/42

(30) Priority: 15.02.90 GB 9003430

(43) Date of publication of application:
21.08.91 Bulletin 91/34

(84) Designated Contracting States:
AT BE CH DE DK ES FR GB GR IT LI LU NL SE

(71) Applicant: JOHNSON MATTHEY PUBLIC
LIMITED COMPANY
78 Hatton Garden
London, EC1N 8JP(GB)

(72) Inventor: Savage, Paul David
1 Thetford Mews, Caversham Park Village
Reading, RG4 0FN(GB)
Inventor: Theobald, Brian Ronald Charles
15 Lea Road, Sonning Common
Reading, RG4 9LH(GB)

(74) Representative: Wishart, Ian Carmichael et al
Patents Department Johnson Matthey
Technology Centre Blounts Court Sonning
Common
Reading, Berks RG4 9NH(GB)

(54) **Improvements in chemical compounds.**

(57) Certain polyoxotungstate ions, having additional atoms selected from P, transition metals and group 13 metals, and which have a metal cluster structure which is not the Keggin structure, form pharmaceutical compositions with anti-viral, especially anti-HIV, activity. Examples of compounds include those with ions of formula a),

$$[P_2H_cW_{12}M'_xO_y]^{p-}$$

where M' is a transition metal, x is 0 or an integer from 1 to 6, y is an integer from 48 to 62, c is 0, 1 or 2, and p is an integer dependent upon the oxidation state of the transition metal M'.

EP 0 442 663 A1

This invention concerns improvements in chemical compounds, more especially it concerns compounds and pharmaceutical compositions. In particular, it concerns compositions and compounds having activity in in vitro tests on Human Immunodeficiency Virus-infected cells.

The disease known as Acquired Immune Deficiency Syndrome (AIDS) caused by infection by HIV has attracted immense research effort because of the effects of the disease on infected individuals and the dangers of the disease spreading to a wider section of the population. In general, although various chemotherapeutic treatments have been advocated, and some compounds have emerged as a potential basis for treatment, there is still a need for alternatives. In particular, most treatments such as the compound known as AZT have a high toxicity to cells, and it would be desirable to find compounds which are less toxic. In man, the development of resistance to AZT has been identified as an additional clinical problem.

We have found certain compounds which show protective properties in in vitro screens of cells challenged with HIV-1 and/or HIV-2, and are therefore indicated as having potential for the treatment of AIDS and AIDS Related Complex and other viral and especially retroviral infections. Accordingly, the present invention provides the use of compounds defined below, in pharmaceutical compositions for treating HIV-infected patients. The invention further provides pharmaceutical compositions comprising a said compound in combination with a pharmaceutically acceptable diluent or carrier, for the treatment of HIV-infected patients. The invention may also be defined as the use of a said compound for the manufacture of a medicament for the treatment of mammals infected with or challenged by, a viral infection, especially HIV-infected patients. The invention further provides a process for the production of a pharmaceutical composition for the treatment of a HIV-infected patient, comprising the combination of a compound as defined below with a pharmaceutically acceptable diluent or excipient, and formulating said composition into a form suitable for administration to said patient. The invention also provides a method of treatment of an HIV-infected patient, comprising administering to said patient an effective dose of a said compound. It is to be understood that treatment includes prophylactic treatment of patients at risk, in view of the protective properties observed. Whilst this description is especially directed to combating HIV, this invention includes other aspects in which other diseases may be treated, including for example microbial infections.

It has been suggested, in JP 64-38,022, that certain salts of heteropolyacid ions of general formula $(XM_{12}O_{40})^{p-}$ where X is an ion selected from Groups III to VI or transition metal, M is one to three species selected from Mo, W, Al, V, Nb, Ta, Co and Ti, and P is a positive integer, exemplified by the two compounds $K_5BW_{12}O_{40}$ and $K_7PW_{10}Ti_2O_{40}$, which have activity against herpes virus, could be expected to have activity against human retrovirus. All the compounds have a structure of the type known as the "Keggin" structure.

It has also been reported in Chemical and Engineering News, December 1986, that silicotungstate acid, $H_4SiW_{12}O_{40}$ has activity against HIV; this compound has, however, been abandoned because of its toxicity in higher animals. This compound also has the Keggin structure.

We have now discovered certain polyoxometalate compounds of different type, which exhibit very significant activity in conventional screening trials. Accordingly, the present invention provides as active compound, a substantially non-toxic ionic polyoxotungstate compound, said compound having a polyoxotungstate ion with a metal cluster structure other than the Keggin structure and which ion contains at least one atom from phosphorus and transition and Group 13 metals, selected from the ions:

a) $[P_2H_cW_{12}M'_xO_y]^{p-}$, in which
M' is a transition metal, x is 0 or an integer from 1 to 6, y is an integer from 48 to 62, c is 0, 1 or 2, and p is an integer dependent upon the oxidation state of the transition metal M',

b) $[P_2W_{18}M'_4(H_2O)_2 O_{68}]^{p-}$, in which
M' is a transition metal, and p is an integer dependent upon the oxidation state of the transition metal M',

c) $[M''W_{10}O_{35}]^{p-}$, in which
M'' is a lanthanide series metal and p is an integer dependent upon the oxidation state of the lanthanide metal M'',

d) the reaction product of silicotungstate ion and $PtX_2(PR_3)_2$ in which X is a co-ordinating anionic ligand or $X_2$ is a chelating anionic ligand, and R is an alkyl or aryl group, or the $[Pt(OH_2)_2L_2]^{2+}$ ion in which each L is an amine or ammine or $L_2$ is a chelating amine, or the product of the phosphotungstate ion and said $[Pt(OH_2)_2L_2]^{2+}$ ion,

e) $[P_2W_{18-a}M'''_aO_{62-b}]^{p-}$, in which
a is 0 or a positive integer from 1 to 5, b is 0 or an integer which depends on the value of a, M''' is a metal from Groups 5 to 13 and p is a positive integer which is dependant upon the value of a, and

f) $[P_4W_{14}O_{58}]^{12-}$.

Herein, references to Groups of the Periodic Table are made in agreement with the Periodic Table published in the 69th Edition of the CRC Handbook of Chemistry and Physics (1988).

In the case of the reaction products e) above, the structures and the molecular formulae of the products have not yet been clarified. In all cases, we believe that the ionic structures are not necessarily maintained in solution, especially as pH changes, and it is not possible to ascertain at this time what structures are active when in contact with living cells. In particular, the structures may lose metal atoms and oxygen atoms, that is, parts of the molecule may split off yet the molecule may remain active in the antiviral tests. Eventually, of course, if exposed to relatively high or low pH's or to other conditions which are relatively severe, degradation of the molecule will result in much simpler molecular fragments which are inactive. The invention is intended to encompass all aqua complexes and molecular fragments of the above compounds which retain a desirable level of anti-viral activity. It is to be understood that active molecular fragments may be administered or included in pharmaceutical compositions as such, or may be made available in a number of different ways.

It is not believed that any of the above active compounds and molecular fragments have been previously proposed as having activity against retroviruses such as HIV, and the above-mentioned JP 64-38,022 does not suggest or yield in practice the active compounds and fragments of the present invention.

The skilled man will readily appreciate that a molecular structure does not exist for every value of x and y in ion a), for example; examples of active structures will be given hereinafter.

The active compounds of the present invention are ionic, and exist with one or more suitable counter-ions. As counter-ions may be considered especially hydrogen, alkali metals and alkaline earth metals, ammonium and substituted ammonium for example substituted by one, two, three or four alkyl or aryl groups and other basic nitrogen-containing organic compounds such as amino acids. It is not believed that the counter-ion is especially critical to activity, although it has an influence on toxicity.

The ions of groups a) to c), e) and f) are known per se and described in the literature. The reaction product d) is considered to be novel and hence forms part of the present invention as such. The reactions may be carried out by mixing solutions of the reactants and heating, then recovering the product using conventional techniques. The processes are illustrated in the following preparative examples.

EXAMPLES 1 (reaction product d)

A solution of $[PtCl_2(PPh_3)_2]$ (285mg) in 10ml diethyl ether was added to a solution of 500mg $H_4SiW_{12}O_{40}$ in 15ml of diethylether, and the reaction mixture was heated under reflux for 24 hours. Over this period, a cream-yellow oil was obtained, which was then separated by decantation and continuously triturated with 50ml of petroleum ether until a solid was obtained. The solid was collected by filtration, washed with ethanol and diethylether and then air-dried. The crude sample was recrystallised three times from an acetone/diethylketone ether mixture to obtain a cream-yellow powder (254mg).

EXAMPLE 2 (reaction product d)

A solution of 240mg of $[PtCl_2(PPh_2Me)_2]$ in 10ml of diethylether was reacted with 500mg of $H_4SiW_{12}O_{40}$ dissolved in 15ml of diethylether, using identical conditions to those described in Example 1. The recrystallised product was a lustrous cream powder (196mg).

The products of Examples 1 and 2 were examined by IR spectroscopy and the results are shown in Table 1 below, and the elemental analyses are shown in Table 2.

3

## TABLE 1

IR stretching frequencies in $cm^{-1}$

| Ex1 | Ex2 | assignments |
|---|---|---|
| 1470 | 1480 | $\nu(P-Ph)$ |
| 1420 | 1430 | |
| 1010 | 1015 | $\nu_s W-O_d$ |
| 980 | 978 | $\rho\nu_{as} W-O_d$ |
| 920 | 910 | $\nu_{as} Si-O_a$ |
| 830 | 860 | $\nu_{as} W-O_b-W$ |
| 750 | 800 | $\nu_{as} W-O_d-W$ |
| 730 | 750 | |
| 680 | 690 | |
| 550 | 525 | $\rho W-O-W/$ |
| 525 | 520 | $\rho O-Si-O$ |
| 340 | 335 | $\rho W-O-W$ |
| 320 | 310 | |

## TABLE 2

The analytical data is calculated in wt% W, Pt, P, C, H.

| Compound | W | Pt | P | C | H |
|---|---|---|---|---|---|
| Ex 1 | 48.53 | 6.90 | 2.52 | 15.6 | 1.6 |
| Ex 2 | 46.43 | 5.82 | 2.86 | 14.05 | 1.6 |

The absence of bands attributable to Pt-Cl stretch at ca 200cm$^{-1}$ indicated that no chloride ligands were retained in the product compounds. It is suggested that the actual structure may be either two Keggin ion fragments bonding to three Pt-phosphine moieties, or a trigonal arrangement of Pt-phosphine moieties surrounded by two heteropolyanions.

EXAMPLE 3 (reaction product d)

500mg of $H_4SiW_{12}O_{40}$ was dissolved in 25ml of warm water and 19mg of sodium carbonate was added. An aqueous solution of 149mg of $[Pt(OH_2)_2(H_2NC_2H_4NH_2)]2NO_3$ was added dropwise until the reaction mixture became cloudy. The solution was gently heated under reflux for 4 to 6 hours, and allowed to cool overnight. Large yellow green crystals were obtained which were collected by filtration, washed with water and with ethanol and then air-dried (228mg).

EXAMPLE 4 (reaction product d)

500mg of $H_3PW_{12}O_{40}$ was dissolved in warm water and 19mg of sodium carbonate was added. A solution of 149mg of $[Pt(OH_2)_2(H_2NC_2H_4NH_2)]2NO_3$ in water was added, and a flocculent yellow precipitate formed immediately.

The reaction mixture was gently refluxed for 12 hours and the yellow powder was recrystallised three times from a warm water/methanol mixture. The powder was collected by filtration, washed with water, with ethanol and with diethyl ether, and then dried in vacuo (173mg).

The reaction products of Examples 3 and 4 were studied by infra-red spectroscopy and by elemental analysis, and the results are shown in Tables 3 and 4 below.

## TABLE 3

IR stretching frequencies in $cm^{-1}$.

| Ex3 | Ex4 | assignments |
|-----|-----|-------------|
| 3250 | 3200 | $\nu(-NH_2)$ |
| 3200 | 3180 | |
| 1625 | 1600 | $\delta(-NH_2)$ |
| 1560 | 1560 | |
| | 1075 | $\nu_{as}P-O_a$ |
| | 1040 | |
| 1010 | | $\rho\nu_a W-O_d$ |
| 975 | 965 | $\nu_{as}W-O_d$ |
| 920 | | $\nu_{as}Si-O_a$ |
| 875 | 890 | $\nu_{as}W-O_b-W$ |
| | 865 | |
| 810 | 825 | $\nu_{as}W-O_c-W$ |
| 720 | 720 | |
| 525 | 535 | $\delta W-O-W/$ |
| | | $\delta O-Si-O$ |
| 375 | 360 | $\delta W-O-W$ |
| 330 | 340 | |

## TABLE 4

|      | W     | Pt    | P    | C    | H    | N    |
|------|-------|-------|------|------|------|------|
| Ex3  | 52.55 | 10.57 | –    | 1.36 | 1.24 | 1.45 |
| Ex4  | 45.89 | 11.25 | 0.96 | 1.40 | 1.02 | 1.50 |

The IR spectra show diamine and polyoxoanion stretching frequencies.

The compounds of the invention were tested in a screen by the MTT method (J.Virol. Methods 120: 309-321 [1988]). MT-4 cells ($2.5 \times 10^4$ / well) were challenged with HIV-1 (HTLV-IIIB) or HIV-2 (LAV-2 ROD) at a concentration of 100 $CCID_{50}$ and incubated in the presence of various concentrations of the test compounds, which were added immediately after challenge with the virus. After 5 days culture at 37°C in a $CO_2$ incubator, the number of viable cells was assessed by the MTT (tetrazolium) method. Antiviral activity and cytotoxicity of the compounds are expressed in the table below as $ED_{50}$ (ug/ml) and $CD_{50}$ (ug/ml), respectively. The potential therapeutic usefulness was assessed by calculating a Selectivity Index (SI) corresponding to the ratio of $CD_{50}$ to $ED_{50}$. A control test was performed using the known anti-HIV treatment AZT, and a comparison compound HPA-23 was also run through the screen as detailed below.

In the table below, the compounds screened were

| A | $K_{12}[H_2P_2W_{12}O_{48}]$ | ion type a) |
|---|---|---|
| B | $K_{10}[P_2W_{18}Zn_4(H_2O)_2O_{68}]$ | ion type b) |
| C | $Na_7[PrW_{10}O_{35}]$ | ion type c) |
| D | Ex1 Product | ion type d) |
| E | $K_9Li[P_2W_{17}O_{62}]$ | ion type e) |
| F | $Na_{12}[P_4W_{14}O_{58}]$ | ion type f) |
| G | Ex 3 Product | ion type d) |
| H | $K_{10}[P_2W_{18}Co_4(H_2O)_2O_{68}]$ | ion type b) |
| I | Ex 4 Product | ion type d) |

For comparison, AZT (azidothymidione) and HPA-23 (the heteropolyanion $(NH_4)_{17}H[NaSb_9W_{21}O_{86}]$ were subjected to the identical tests, and the results obtained are also listed.

## TABLE 5

| Compound | Virus | $CD_{50}$ | $ED_{50}$ | SI |
|---|---|---|---|---|
| A | HIV-1 | 721.0 | 0.089 | 8138 |
|   | HIV-2 | 675.9 | 0.153 | 4403 |
| B | HIV-1 | 466.3 | 0.71 | 661 |
|   | HIV-2 | 441.9 | 18.03 | 25 |
| C | HIV-1 | 761.8 | 1.86 | 409 |
|   | HIV-2 | 221.7 | 2.70 | 285 |
| D | HIV-1 | 1000.0 | 4.57 | 219 |
|   | HIV-2 | 1000.0 | 19.63 | 51 |
| E | HIV-1 | 502.9 | 2.92 | 172 |
|   | HIV-2 | 429.7 | 7.28 | 59 |
| F | HIV-1 | 655.8 | 4.82 | 136 |
|   | HIV-2 | 682.8 | 13.26 | 51 |
| G | HIV-1 | 477.0 | 3.81 | 125 |
|   | HIV-2 | 573.9 | 12.90 | 44 |

| | | | | | |
|---|---|---|---|---|---|
| H | | HIV-1 | 84.8 | 1.47 | 58 |
| | | HIV-2 | 81.6 | 12.26 | 7 |
| I | | HIV-1 | 4.1 | 0.086 | 48 |
| | | HIV-2 | 11.0 | 40.0 | <1 |
| AZT | | HIV-1 | >1 | <0.008 | >125 |
| HPA 23 | | HIV-1 | 7.4 | 2.8 | <2.7 |
| | | HIV-2 | 2.4 | >4 | <1 |

In this field of study, it is considered that any compound exhibiting a Selectivity Index of greater than 5 has the potential for further study. The compounds of the present invention show an appreciably greater selectivity index than the antimony-tungsten rodate compound HPA-23.

The following preparative examples illustrate additional compounds. In all cases, the compounds were tested against HIV-1 and HIV-2 using the same procedure as above. Duplicate tests were performed and both sets of results are given.

EXAMPLE 5 (Ion type a)

$K_{12}H_6W_{12}Ti_6O_{62}$ 31$H_2O$
$K_{12}[H_2P_2W_{12}O_{48}].24H_2O$ (10g, 2.5mmol) was dissolved in 1M LiCl (125ml) and 1M TiCl$_4$ in dichloromethane (15ml, 0.015mol) was added slowly. A white precipitate formed immediately. The mixture was stirred for 2 hours, collected by filtration and dried in vacuo. This material was dissolved in water (120ml) at 50°C, filtered and KCl (15g) added to the cooled filtrate. The copious white precipitate was collected by filtration and dried in vacuo.
    Yield = 8.4g

| | | K | P | W | Ti |
|---|---|---|---|---|---|
| $K_{12}H_6P_2W_{12}Ti_6O_{62}.31H_2O$ | calc | 10.24 | 1.35 | 48.17 | 6.27 |
| | found | 10.85 | 1.45 | 48.26 | 6.41 |

| | CD$_{50}$ | ED$_{50}$ | SI |
|---|---|---|---|
| HIV-1 | 201.0 | 10.1 | 20 |
| HIV-1 | 185.0 | 5.4 | 34 |
| HIV-2 | 243.0 | 7.0 | 42 |
| HIV-2 | 172.0 | 6.4 | 27 |

EXAMPLE 6 (ion type a)

$K_2(\text{arginium})_{10}[H_2P_2W_{12}O_{48}].5H_2O$

$K_{12}[H_2P_2W_{12}O_{48}]24H_2O$ (3.94g, 1mmol) was suspended in water (30ml) and warmed to 35°C. Arginine hydrochloride (10g, 0.047mol) was dissolved in water (15ml) and added to the tungstate solution. The clear solution was put in a refrigerator and left until crystallisation was complete. The product was collected by filtration and dried in vacuo.

Yield = 1.7g.

|  |  | C | H | N | P |
|---|---|---|---|---|---|
| $C_{60}H_{160}N_{40}O_{73}P_2W_{12}K_2$ | calc | 14.53 | 3.23 | 11.30 | 1.25 |
|  | found | 14.78 | 3.58 | 10.80 | 1.26 |

|  | $CD_{50}$ | $ED_{50}$ | SI |
|---|---|---|---|
| HIV-1 | >500 | 2.83 | >179 |
| HIV-1 | >500 | 7.3 | > 69 |
| HIV-2 | >500 | 39.6 | > 13 |
| HIV-2 | >500 | 3.4 | >148 |

EXAMPLE 7 (ion type a))

$(NH_4)_{12}[H_2P_2W_{12}O_{48}].16H_2O$

$K_{12}[H_2P_2W_{12}O_{48}]24H_2O$ (3.94g, 1mmol) was suspended in water (30ml) and warmed to 35°C. NH₄Cl (10g, 0.19mol) was dissolved in water (35ml) and added to the tungstate solution. An oil separated and the supernatant liquid was decanted off. The oil was extracted twice with a little water, decanted off, and the oil crystallised by the addition of ethanol. The white solid was collected by filtration, washed with ethanol and dried on the filter. This compound was redissolved in water (40ml) at 40°C and allowed to cool. NH₄Cl (4.5g) in water (15ml) was added in 1ml portions with stirring. The resulting white precipitate was collected by filtration, washed with water and ethanol and dried in vacuo.

Yield = 2.1g

|  |  | P | W |
|---|---|---|---|
| $H_{50}N_{12}P_2W_{12}O_{48}.16H_2O$ | calc | 1.75 | 62.24 |
|  | found | 1.94 | 62.13 |

|  | $CD_{50}$ | $ED_{50}$ | SI |
|---|---|---|---|
| HIV-1 | >500.0 | 2.9 | >171 |
| HIV-1 | 304.0 | 1.29 | 236 |
| HIV-2 | 346.0 | 5.3 | 65 |
| HIV-2 | 286.0 | 1.6 | 181 |

EXAMPLE 8 (ion type a)

$K_8$(histidinium)$[H_2P_2W_{12}O_{48}]$
The method used in Example 6 was followed except that histidinium hydrochloride (5g, 0.026mol) in water (10ml) was used instead of arginine hydrochloride.
Yield = 3.7g

| | | C | H | N | P |
|---|---|---|---|---|---|
| $C_{24}H_{42}N_{12}O_{56}K_8P_2W_{12}$ | calc | 7.25 | 1.06 | 4.23 | 1.56 |
| | found | 7.55 | 1.41 | 4.30 | 1.78 |

| | $CD_{50}$ | $ED_{50}$ | SI |
|---|---|---|---|
| HIV-1 | >500 | 1.9 | >263 |
| HIV-1 | >500 | 12.0 | >42 |
| HIV-2 | >500 | >500 | 1 |
| HIV-2 | >500 | 4.3 | >117 |

EXAMPLE 9 (ion type a))

$K_{8.5}$(lysinium)$_{3.5}[H_2P_2W_{12}O_{48}]$
The method of Example 6 was followed, except that lysinium hydrochloride (5g, 0.027mol) in water (10ml) was used instead of arginine hydrochloride.
Yield = 3.2g

| | | C | H | N | P |
|---|---|---|---|---|---|
| $C_{21}H_{52.5}N_7O_{55}P_2W_{12}K_{8.5}$ | calc | 6.49 | 1.35 | 2.52 | 1.60 |
| | found | 6.42 | 1.62 | 2.41 | 1.88 |

| | $CD_{50}$ | $ED_{50}$ | SI |
|---|---|---|---|
| HIV-1 | 190.0 | 3.3 | 59 |
| HIV-1 | 127.0 | 3.0 | 43 |
| HIV-2 | 253.0 | 2.8 | 92 |
| HIV-2 | 128.0 | 1.5 | 84 |

EXAMPLE 10 (ion type a)

$K_4$(cyclam)$_4[H_2P_2W_{12}O_{48}]$
The method of Example 6 was followed except that cyclam hydrochloride (5g, 0.018mol) in water (10ml) was used.
Yield = 0.34g

|  | | C | H | N | P |
|---|---|---|---|---|---|
| $C_{40}H_{104}N_{16}O_{48}P_2W_{12}K_4$ | calc | 12.00 | 2.60 | 5.60 | 1.55 |
|  | found | 12.66 | 3.00 | 5.78 | 1.42 |

|  | $CD_{50}$ | $ED_{50}$ | SI |
|---|---|---|---|
| HIV-1 | 314.0 | 11.0 | 29 |
| HIV-1 | >500 | 22.2 | >23 |
| HIV-2 | >500 | 5.5 | >90 |
| HIV-2 | >500 | 37.7 | >13 |

EXAMPLE 11 (ion type a)

$K_6[P_2W_{12}Mo_6O_{62}]$

The method described in R Contant and J-P Ciabrini, J Chem Res, (1977) 2601-2609 was followed to yield the above-identified compound.

|  | $CD_{50}$ | $ED_{50}$ | SI |
|---|---|---|---|
| HIV-1 | 37.0 | 1.2 | 30 |
| HIV-1 | 114.0 | 1.3 | 88 |
| HIV-2 | 343.0 | 1.14 | 300 |
| HIV-2 | 159.0 | 2.6 | 60 |

EXAMPLE 12 (ion type a)

Reduced form of $K_6[P_2W_{12}Mo_6O_{62}]$

$K_{12}[H_2P_2W_{12}O_{48}].24H_2O$ (20g) was dissolved in 1M LiCl (250ml) acidified with 1M HCl (5ml) and 1M $Li_2Mo_4$ (30ml) added. 1M HCl (60ml) was then added dropwise. The final pH was ~5. A further portion of 1M HCl (50ml) was added followed by KI (50g). The resulting deep blue crystalline material was collected by filtration, washed with ethanol and dried in vacuo. The product is $K_xH_y[P_2W_{12}Mo_6O_{62}]$, where x and y could not be established.

Yield = 14.0g

|  | $CD_{50}$ | $ED_{50}$ | SI |
|---|---|---|---|
| HIV-1 | 234.0 | 4.1 | 57 |
| HIV-1 | 300.0 | 5.8 | 52 |
| HIV-2 | 315.0 | 1.8 | 173 |
| HIV-2 | 306.0 | 1.4 | 214 |

EXAMPLE 13 (ion type c)

$K_6[CeW_{10}O_{35}].18H_2O$
The method described by R D Peacock and T J R Weakley, J Chem Soc A (1971), 1836, was followed to obtain the above-identified compound. Its composition was confirmed by elemental analysis.

|  | $CD_{50}$ | $ED_{50}$ | SI |
|---|---|---|---|
| HIV-1 | 312 | >500 | <1 |
| HIV-1 | 299 | 85.4 | 4 |
| HIV-2 | 255 | 76.5 | 3 |
| HIV-2 | 282 | 50.2 | 5 |

EXAMPLE 14 (ion type c)

$K_7[GdW_{10}O_{35}].18H_2O$
The method described in the literature reference identified in Example 13 was followed to form the analogous compound $K_7[GdW_{10}O_{35}].18H_2O$. Its composition was confirmed by elemental analysis.

|  | $CD_{50}$ | $ED_{50}$ | SI |
|---|---|---|---|
| HIV-1 | 308.0 | 6.8 | 45 |
| HIV-1 | 272.0 | 19.1 | 14 |
| HIV-2 | 335.0 | 3.48 | 96 |
| HIV-2 | 233.0 | 200 | 12 |

EXAMPLE 15 (ion type c)

$K_7[ErW_{10}O_{35}].22H_2O$
The method described in Example 13 was followed to form the analogous compound $K_7[ErW_{10}O_{35}].22H_2O$. Its composition was confirmed by elemental analysis.

|  | $CD_{50}$ | $ED_{50}$ | SI |
|---|---|---|---|
| HIV-1 | 253.0 | 45.7 | 6 |
| HIV-1 | 270.0 | 4.95 | 55 |
| HIV-2 | 210.0 | 41.1 | 5 |
| HIV-2 | 318.0 | 9.6 | 3 |

EXAMPLE 16 (ion type e)
$K_6P_2W_{15}Mo_3O_{62}.11H_2O$ was prepared according to R Contant and J P Ciabrini, J Chem Res (M),(1977), 2610-2618

13

|         | $CD_{50}$ | $ED_{50}$ | SI  |
|---------|-----------|-----------|-----|
| HIV-1   | 83.0      | 0.49      | 168 |
| HIV-1   | 500.0     | 5.0       | 99  |
| HIV-2   | 236.0     | >500      | <1  |
| HIV-2   | 500.0     | 10.6      | 47  |

EXAMPLE 17 (ion type e)

$K_3HP_2W_{15}V_3O_{62}.34H_2O$ was prepared according to R G Finke et al, J Am Chem Soc,(1986), 108, 2947-2960

|         | $CD_{50}$ | $ED_{50}$ | SI     |
|---------|-----------|-----------|--------|
| HIV-1   | >500      | 0.46      | >1098  |
| HIV-1   | 307       | 5.1       | 60     |
| HIV-2   | 168       | 7.1       | 24     |
| HIV-2   | 154       | `7.4      | 21     |

EXAMPLE 18

$Co(NO_3)_2.6H_2O$ (0.96g, 3.3mmol) was dissolved in water (50ml) followed by 6M HCl (1.6ml, 0.96mmol). To the resulting solution $Na_{12}P_2W_{15}O_{56}.18H_2O$ (4.6g, 1.1mmol) was slowly added. The mixture was stirred for 10 minutes and the product was precipitated by adding KCl (10g). The product was collected by filtration and recrystallised from dilute hydrochloric acid (pH ~1.5, 35ml) at 80°C. The compound was collected by filtration and dried in vacuo.

|         | K    | P    | Co   | W     |
|---------|------|------|------|-------|
| found   | 5.77 | 1.61 | 1.53 | 62.04 |

The structure of the product could not be determined unambiguously.

|         | $CD_{50}$ | $ED_{50}$ | SI  |
|---------|-----------|-----------|-----|
| HIV-1   | 268.0     | 6.24      | 43  |
| HIV-1   | 179.0     | 3.1       | 59  |
| HIV-2   | 75.0      | 5.3       | 11  |
| HIV-2   | 223.0     | 1.3       | 169 |

EXAMPLE 19

The procedure of Example 18 was followed except that $ZnCl_2$ (0.45g, 3.3mmol) was used instead of Co-$(NO_3)_2.6H_2O$.

14

|  | K | P | W | Zn |
|---|---|---|---|---|
| found | 5.97 | 1.61 | 61.19 | 1.86 |

The structure of the product could not be determined unambiguously.

|  | $CD_{50}$ | $ED_{50}$ | SI |
|---|---|---|---|
| HIV-1 | 44.0 | 3.0 | 15 |
| HIV-1 | 219.0 | 0.7 | 308 |
| HIV-2 | 288.0 | 2.0 | 144 |
| HIV-2 | 175.0 | 5.3 | 33 |

EXAMPLE 20

Reaction product of $Na_{12}P_2W_{15}O_{56}.18H_2O$ and $Al(NO_3)_3.9H_2O$.
The procedure described in Example 18 was followed except that $Al(NO_3)_3.9H_2O$ (1.2g, 3.3mol) was used instead of $Co(NO_3)_2.6H_2O$. Elemental analysis of the product recovered, was carried out:

|  | K | P | W | Al |
|---|---|---|---|---|
| found | 7.88 | 1.93 | 77.39 | 0.39 |

The structure of the product was not able to be determined.

|  | $CD_{50}$ | $ED_{50}$ | SI |
|---|---|---|---|
| HIV-1 | 280 | 8.9 | 32 |
| HIV-1 | 426 | 20.4 | 21 |
| HIV-2 | 310 | 6.7 | 46 |
| HIV-2 | 482 | 15.8 | 31 |

EXAMPLES 21-25 (ion type e)
The following compounds were prepared by routes described in the literature and Selectivity Indices were determined in accordance with the procedures described above.

## EXAMPLE 21

| | SI | |
| --- | --- | --- |
| | HIV-1 | HIV-2 |
| $(NH_4)_6P_2W_{18}O_{62}$ | 301 | 28 |
| | 482 | 88 |

## EXAMPLE 22

| | | |
| --- | --- | --- |
| $K_6P_2W_{17}MoO_{62}$ | 370 | 44 |
| | 30 | 41 |

| | | SI | |
| --- | --- | --- | --- |
| EXAMPLE 23 | | HIV-1 | HIV-2 |
| $Na_{12}P_2W_{16}O_{59}$ | | 250 | 606 |
| | | 228 | 57 |

## EXAMPLE 24 (oligomer ion)

| | | |
| --- | --- | --- |
| $K_{16}Li_2H_6P_4W_{24}O_{94}$ | 670 | 100 |
| | 505 | 634 |

## EXAMPLE 25 (oligomer ion)

| | | |
| --- | --- | --- |
| $K_{28}Li_5H_7P_8W_{48}O_{184}$ | 47 | 41 |
| | 14 | 21 |

The active compounds may be administered in the form of pharmaceutical compositions formulated according to well known principles and incorporating the compound, preferably in unit dose form, in combination with a pharmaceutically acceptable diluent or carrier. Such compositions may be in the form of solutions or suspensions for injection, or irrigation or be in capsule, tablet, dragee, or other solid composition or as a solution or suspension for oral administration or formulated into pessaries or suppositories or sustained release forms of any of the above or for implantation. Suitable diluents, carriers, excipients and other components are known. It may be desirable also to formulate a composition for topical administration such as an ointment or cream. The compounds of the invention may be used, in the form of a composition or alone, and possibly carried on a finely divided support, as a coating on devices which in use contact body fluids, to discourage transmission of viral infections. Examples of devices to be considered in this aspect of the invention are surgical devices and gloves and contraceptives such as condoms, and other items, appliances, wound dressings and coverings, implements etc generally to be considered as devices according to this aspect of the invention.

The pharmaceutical compositions according to the invention may contain unit dosages determined in accordance with conventional pharmacological methods, suitably to provide active compounds in the dosage range in humans of from 0.1 to 100 mg/kg body weight per day, in a single dose or in a number of smaller doses. Preferred dosage ranges are 1 to 30 mg/kg body weight per day.

## Claims

1. A pharmaceutical composition for anti-viral use in mammals comprising a substantially non-toxic ionic polyoxotungstate compound, said compound having a polyoxotungstate ion with a metal cluster structure other than the Keggin structure and which ion contains at least one atom selected from phosphorus and transition and Group 13 metals, selected from the ions:

   a) $[P_2H_cW_{12}M'_xO_y]^{p-}$, in which
   M' is a transition metal, x is 0 or an integer from 1 to 6, y is an integer from 48 to 62, c is 0, 1 or 2 and p is an integer dependent upon the oxidation state of the transition metal M',

   b) $[P_2W_{18}M'_4(H_2O)_2O_{68}]^{p-}$, in which
   M' is a transition metal, and p is an integer dependent upon the oxidation state of the transition metal M',

   c) $[M''W_{10}O_{35}]^{p-}$, in which
   M'' is a lanthanide deries metal and p is an integer dependent upon the oxidation of the lanthanide metal M'',

   d) the reaction product of silicotungstate ion and $PtX_2(PR_3)_2$ in which X is a co-ordinating anionic ligand or $X_2$ is a chelating anionic ligand, and R is an alkyl or aryl group, or the $[Pt(OH_2)_2L_2]^{2+}$ ion in which each L is an amine or ammine or $L_2$ is a chelating amine, or the product of the phosphotungstate ion and said $[Pt(OH_2)_2L_2]^{2+}$ ion,

   e) $[P_2W_{18-a}M'''_aO_{62-b}]^{p-}$, in which
   a is 0 or a positive integer from 1 to 5, b is 0 or an integer which depends on the value of a, M''' is a metal from groups 5 to 13 and p is an integer which is dependent upon the value of a, or an oligomer thereof, and

   f) $[P_4W_{14}O_{58}]^{12-}$,

   in admixture with a pharmaceutically acceptable diluent or carrier.

2. A composition according to claim 1, wherein the polyoxotungstate ion has formula a).

3. A composition according to claim 1, wherein the polyoxotungstate ion has the formula $[P_2W_{12}M'_xO_y]^{p-}$.

4. A composition according to claim 2, wherein the ion is of formula

   $[H_2P_2W_{12}O_{48}]^{12-}$

5. A composition according to claim 1, wherein the polyoxotungstate ion has formula e).

6. A composition according to claim 1, wherein the polyoxotungstate ion has the formula $[P_2W_{18-a}O_{62-b}]^{p-}$.

7. A composition according to claim 5, wherein the ion is of formula

   $[P_2W_{15}V_3O_{62}]^{9-}$

8. A composition according to any one of the preceding claims, in unit dosage form.

9. A composition according to any one of claims 1 to 7, in the form of a composition for topical administration or for coating.

10. An ionic platinum polyoxotungstate material whioh is a reaction product of silicotungstate ion and $PtX_2(PR_3)_2$ in which X is a co-ordinating anionic ligand or $X_2$ is a chelating anionic ligand and R is an alkyl or aryl group, or the silicotungstate or phosphotungstate ion and the ion $[Pt(OH_2)_2L_2]^{2+}$ in which each L is an amine or ammine or $L_2$ is a chelating amine.

11. The use of an ionic polyoxotungstate compound as defined in any one of claims 1 to 10, for the preparation of a medicament for the treatment of a mammal infected with or challenged by, a viral infection.

12. The use of claim 11, in which the viral infection is HIV.

European
Patent Office

EUROPEAN SEARCH
REPORT

Application Number

EP 91 30 0997

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-1 385 489 (AGENCE NATIONALE DE VALORISA-TION DE LA RECHERCHE (ANVAR)) <br> * page 22, line 51 - page 23, line 43; claims 1-26 * <br> – – – | 1-12 | A 61 K 33/24 <br> A 61 K 33/42 |
| E | EP-A-0 388 245 (TERUMO K.K.) <br> * Page 7, line 34 - page 8, line 7; claims 1-11 * <br> – – – | 1-12 | |
| A,P | EP-A-0 390 365 (JOHNSON MATTHEY PUBLIC LTD CO.) <br> – – – – – | 1-12 | |

TECHNICAL FIELDS
SEARCHED (Int. Cl.5)

A 61 K

The present search report has been drawn up for all claims

| Place of search | Date of completion of search | Examiner |
|---|---|---|
| The Hague | 21 May 91 | BRINKMANN C. |